(19) Europäisches Patentamt
European Patent Office
Office européen des brevets

(11) **EP 3 069 651 A1**

(12) **EUROPEAN PATENT APPLICATION**
published in accordance with Art. 153(4) EPC

(43) Date of publication:
**21.09.2016  Bulletin 2016/38**

(51) Int Cl.:
**A61B 3/036** (2006.01)

(21) Application number: **14861549.5**

(22) Date of filing: **18.09.2014**

(86) International application number:
**PCT/CN2014/086791**

(87) International publication number:
**WO 2015/070672 (21.05.2015 Gazette 2015/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **14.11.2013  CN 201310563823**

(71) Applicants:
• **Shenzhen Certainn Technology Co., Ltd**
**Shenzhen, Guangdong 518112 (CN)**
• **Shenzhen Moptim Imaging Technique Co., Ltd.**
**Shenzhen, Guangdong 518000 (CN)**

(72) Inventors:
• **WANG, Hui**
**Shenzhen**
**Guangdong 518112 (CN)**
• **ZHEN, Yi**
**Shenzhen**
**Guangdong 518112 (CN)**
• **GUO, Shuguang**
**Shenzhen**
**Guangdong 518112 (CN)**
• **HE, Weihong**
**Shenzhen**
**Guangdong 518112 (CN)**
• **LUAN, Dun**
**Shenzhen**
**Guangdong 518112 (CN)**
• **LI, Peng**
**Shenzhen**
**Guangdong 518112 (CN)**

(74) Representative: **Johnson, Richard Alan et al**
**Mewburn Ellis LLP**
**City Tower**
**40 Basinghall Street**
**London EC2V 5DE (GB)**

(54) **HAND-HELD VISION DETECTING DEVICE AND VISION DETECTING METHOD**

(57)    Disclosed are a hand-held vision detecting device and a method for using the hand-held vision detecting device to realize various eyesight measurements without wearing glasses and the steps therefor, wherein the hand-held vision detecting device at least comprises: an imaging lens group consisting of at least one imaging lens (16) and an eye chart (4). The cornea (15) of the subject can be located at the focal point at one side f the imaging lens group (16). The center of the eye chart (4) is provided at the other side of the imaging lens group (16) and can move forwards and backwards along the optical path. By using this device, the following functions can be achieved: 1. visual acuity measurement of the naked eye; 2. corrected visual acuity measurements; 3. measurements for detecting the strength of the glasses which should be worn when the corrected visual acuity is 1.0; 4. poor vision and amblyopia vision detection; and 5. astigmatism detection. People can detect vision anytime and anywhere through this device and detection method via simple and easy operations, and the detection results are accurate.

Fig. 1

**Description**

TECHNICAL FIELD

The present invention relates to a technical field of optometry devices, and particularly to a hand-held vision detecting device and a vision detecting method.

BACKGROUND

Currently, eye charts on the market used to measure visual acuity are mostly printed eye charts. The visual acuity measurement is performed only by using the eye chart in an environment having a fixed site, a large space and standard eye chart brightness, though the eye chart has many forms. For the subject of the vision detection, the detection is not taken until it is found that there might be some problems on the visual acuity. Such passive detection usually results in an unpleasant effect on the health and lives. If the passive detection is changed into active detection and people can perform the detection anywhere by themselves without environmental restrictions, the vision protection for them can be greatly ensured.

Patent applications in prior art relating to the hand-held vision detecting device include Patent Application No. 99243321.5 entitled "VISION LENS" and Patent Application No. 201020230301.0 entitled "SIMPLE VISION LENS". As for Patent Application No. 99243321.5, a minifier consisting of an eye lens and an objective lens is adopted in the technical solution thereof. By moving a telescopic sleeve, the vision lens can be adjusted and located at a visual acuity value from 0.1 to 1.5 and the visual acuity value can be shown. Based on the analysis of the application, the scale from 0.1 to 1.5 in the application are not located at the same distance for the eye of the subject. The design principle itself of the application is wrong, because E optotypes having different sizes and opening orientations and representing the visual acuity value from 0.1 to 1.5 in the standard eye chart are located on the same plane, and located at the same horizontal distance for the subject, but not located at the different distance for the eye of the subject. As for Patent Application No. 201020230301.0, the technical features are: comprising a vision lens body consisting of a convex lens, a telescopic rotatable sleeve and a circular paperboard with an optotype, the convex lens being fixed to one end of the telescopic rotatable sleeve, the circular pasteboard with the optotype being fixed to the other end of the telescopic rotatable sleeve. However, in the technical solution of this patent application, there is only one E optotype, which doesn't conform to the E optotypes in the standard eye chart having different sizes and different openings due to different visual acuity values. Furthermore, the abstract of this patent application recites "the telescopic rotatable sleeve is stretched or retracted to change the distance from the convex lens to the circular paperboard with the optotype, thereby changing the size of the optotype", but this may be problematic. Specifically, although the size of the image formed from the optotype with the convex lens is changed by changing the distance from the lens and the paperboard with the optotype, but the applicant didn't realize that the distance from the image to a human eye also changes while the visual angle changes very little, and thus the changing range of the visual acuity value is very small. The sizes of the optotypes are commonly discussed with a hypothesis of the same measurement distance. Moreover, it's confusing whether the visual acuity measured by using the method of this patent application is visual acuity of the naked eye, corrected visual acuity, near visual acuity or distant visual acuity, therefore it is ineffective.

Meanwhile, the structural design of the above two patent applications has a common design irrationality. As we know, the distance between the eye chart and the subject should be fixed correctly during the visual acuity measurements. For example, in existing national standard, the standard distance for the distant visual acuity measurements is 5 meters, and the standard distance for the near visual acuity measurements is 25 cm. The technical solutions of these two patent applications cannot meet the requirement of 5-meter distance between the eye chart and the subject during the visual acuity measurements, and thus cannot meet the national standard of the visual acuity measurement by themselves.

SUMMARY

The present invention provides a hand-held vision detecting device for solving the problems that vision patients cannot detect vision anytime and anywhere due to the site restriction and the vision detecting process is complex during the vision measurement.

The technical solutions of the present invention are as follows:

A hand-held vision detecting device comprises an eye chart and an imaging lens group consisting of at least one imaging lens, wherein the cornea of a subject is located on the optical axis at one side of the imaging lens group, and the center of the eye chart is movably provided on the optical axis at the other side of the imaging lens group and can move forwards and backwards along the optical axis.

Moreover, the cornea of the subject is located at the focal point at one side of the imaging lens group.

Moreover, the center of the eye chart is movably provided at the focal point at the other side of the imaging lens group.

Moreover, the hand-held vision detecting device further comprises a sleeve provided in the middle of one end thereof and an adjusting knob in which the sleeve is sheathed, wherein an indicative line is marked on the outer circumference of the sleeve; the eye chart is provided inside of the sleeve and can move away from or close to the imaging lens group along with the sleeve during the rotation of the adjusting knob; a scale mark indicating a diopter value is zero is marked on the outer circumference of the adjusting knob, or the scale mark indicating the diopter value is zero is marked on the outer circumference of the sleeve along its axis; the eye chart is just located at the focal point at the other side of the imaging lens group when the diopter value is zero.

Moreover, scale marks indicating other diopter values D1 are marked on the outer circumference of the adjusting knob or the outer circumference of the sleeve along the axis of the sleeve, and D1 meets the formula: $D1 = x / (f_0 * f_0)$,

Wherein x is a distance from the eye chart to the focal point at the other side of the imaging lens group, and x is positive if the eye chart is away from the imaging lens group, and is negative if the eye chart is close to the imaging lens group.

Moreover, the hand-held vision detecting device further comprises an eye chart fixing base and a eye chart cover, wherein the eye chart fixing base is fixedly provided on the interior side wall of the sleeve; the eye chart is provided between the eye chart fixing base and the eye chart cover; a through hole through which illuminating light or natural light can pass is provided in the middle of the eye chart fixing base and the eye chart cover.

Moreover, the hand-held vision detecting device further comprises an illuminating light source and a battery support which are fixedly provided in sequence inside of the sleeve and behind the eye chart cover, wherein a button panel component and a button are provided in sequence behind the battery support; the button panel component and the button are provided inside of a battery fixing base; the battery fixing base is detachably connected to the sleeve.

Moreover, the hand-held vision detecting device further comprises a handle provided in the middle thereof, wherein the handle is hollow, and two ends of the handle are detachably connected to the adjusting knob and an imaging lens group fixing base, respectively; the imaging lens group is fixed inside of the imaging lens group fixing base.

Moreover, an eye protecting ring is connected to an outer end of the imaging lens group fixing base.

Moreover, the imaging lens group is a single lens, a doublet lens or a lens group.

Moreover, the eye chart is at least a common eye chart.

Moreover, the common eye chart is at least a tumbling E chart, a tumbling C chart or a tumbling letter chart.

Moreover, the eye chart is further provided with an astigmatism chart.

The present invention further provides a method for detecting or correct visual acuity with a hand-held vision detecting device, the technical solutions are as follows:

A vision detecting method for detecting the visual acuity of naked eye with a hand-held vision detecting device, comprising:

aligning the location of a scale mark indicating zero on an adjusting knob with an indicative line;
keeping the location of the scale mark indicating zero stationary, wherein the visual acuity value marked at both sides of the smallest optotypes which can be identified by a subject and the width of strokes of which is equal to the product of the focal length $f_0$ of an imaging lens group and a visual angle $\alpha$ is the visual acuity of the naked eye.

A vision detecting method for detecting corrected visual acuity with a hand-held vision detecting device, comprising:

rotating a adjusting knob until an eye chart being watching is clearest, wherein the visual acuity value marked at both sides of the smallest optotypes which can be identified and the width of strokes of which is equal to the product of the focal length $f_0$ of an imaging lens group and a visual angle $\alpha$ is the best corrected visual acuity;
determining a diopter scale mark corresponding to an indicative line, wherein a diopter value corresponding to the scale mark is the strength of the glasses which should be worn by the subject.

A vision detecting method for detecting poor vision with a hand-held vision detecting device, comprising:

a subject is judged as a patient with poor vision if optotypes in an eye chart which are corresponding to the visual acuity value of 0.3 and the width of strokes of which is equal to the product of the focal length $f_0$ of an imaging lens group and a visual angle $\alpha$ cannot be identified by the subject no matter how an adjusting knob is rotated clockwise or anticlockwise.

A vision detecting method for detecting amblyopia vision with a hand-held vision detecting device, comprising:

a subject is judged as a patient with amblyopia vision if optotypes in an eye chart which are corresponding to the visual acuity value of 1.0 and the width of strokes of which is equal to the product of the focal length $f_0$ of an imaging lens group and a visual angle $\alpha$ cannot be identified by the subject no matter how an adjusting knob is rotated clockwise or anticlockwise by the subject.

The technical effects of the present invention are as follows. 1. Vision detection may be performed anytime and anywhere with the present device, and the device is easy to carry, convenient to use without site and time restrictions. 2. Visual acuity measurements of the naked eye may be performed with the present device. When performing the visual acuity of the naked eye, only the scale mark indicating zero on the adjusting knob is aligned with the indicative line on the sleeve, and the visual acuity value marked at both sides of the smallest E optotypes which can be seen clearly by the naked eye is the visual acuity of the naked eye. 3. Corrected visual acuity measurements may be performed with the present device, and the strength due to the measurement result directly serves as the strength of the glasses which should be worn. The specific corrected visual acuity measuring method is: when the subject watches the eye chart, the adjusting knob is rotated until the smallest E optotypes can be identified by the subject, and at this moment, the visual acuity value marked at both sides of the smallest E optotypes is the best corrected visual acuity value. The diopter value corresponding to the indicative line is the corrected visual acuity value of the subject. 4. Measurements for detecting the strength of the glasses which should be worn when the corrected visual acuity value is 1.0 may be performed with the present device. The adjusting knob is rotated until the device is extended to the maximal length, and then the adjusting knob is rotated slowly anticlockwise until the opening orientations of the E optotypes corresponding to the visual acuity value of 1.0 can be identified and the opening orientations of the E optotypes corresponding to the visual acuity value of 1.2 cannot be identified. At this moment, the diopter value on the adjusting knob pointed to by the indicative line is the strength of the glasses which should be worn when the corrected visual acuity value is 1.0. 5. Poor vision and amblyopia vision measurements may be performed with the present device. The patient who cannot identify the opening orientations of the E optotypes in the eye chart corresponding to the visual acuity value of 0.3 no matter how the adjusting knob is rotated is a patient with poor vision. The patient who cannot identify the opening orientations of the E optotypes in the eye chart corresponding to the visual acuity value of 1.0, but can identify the opening orientations of the E optotypes corresponding to the visual acuity value of 0.8 at most no matter how the adjusting knob is rotated is a patient with amblyopia vision.

BRIEF DESCRIPTION OF DRAWINGS

FIG. 1 is a schematic view of the optical path of the hand-held vision detecting device;
FIG. 2 is a schematic view of the internal structure of the hand-held vision detecting device;
FIG. 3 is an enlarged view of a selected part in FIG. 2;
FIG. 4 is a schematic view showing an indicative line 71 on a sleeve 7 points to a diopter scale mark on an adjusting knob 5;
FIG. 5 is a left view of FIG. 4;
FIG. 6 is a partial view of a tumbling E chart;
FIG. 7 is an eye chart with a peripheral astigmatic chart and an inner partial tumbling E chart;
FIG. 8 illustrates that an angle subtended by the width of a stroke at the center of a lens is equal to a visual angle of a human eye;
FIG. 9 shows a situation that the diopter scale is marked on the sleeve 7.

In the drawings, reference numbers and names of parts are respectively: 1. eye protecting ring; 2. imaging lens group fixing base; 3. handle; 4. eye chart; 5. adjusting knob; 6. illuminating light source; 7. sleeve; 8. battery fixing base; 9. button; 10. component of button; 11. battery group; 12. battery support; 13. eye chart cover; 14. eye chart fixing base; 15. human eye; 16. imaging lens group; 71. indicative line

DETAILED DESCRIPTION

In order to make the technical problem to be solved by the present invention, technical solutions and beneficial effects more clearly understood, in conjunction with the accompanying drawings and the following embodiments, a more detailed description for the present invention is provided hereinafter. It should be understood that the embodiments described herein are only used to explain the present invention and are not intended to limit the present invention.

Two unique aspects when using a hand-held vision detecting device are firstly explained below.

1. The cornea of a subject should be located at the focal point of an imaging lens group when the hand-held detecting device is used to detect the vision of the subject.

As we know, it is stipulated that the line of sight of the subject should be aligned with the line of the visual acuity value 1.0 and the distance between the eye chart and the subject should be 5 meters during the vision detection of the human eye. One eye should be covered before the detection, and the other single eye identifies the opening orientations of E optotypes from top to bottom until it cannot identify. In fact, the visual acuity refers to the ability of the retina to identify images. The visual acuity can be judged with the ability of the retina to identify images, but the eye visual acuity will decrease when the dioptric media of the eye (such as cornea, crystalline lens and vitreous body) becomes opaque or the subject has ametropia (including myopia, hyperopia and astigmatism), even though the retina is fine. The eye chart is only used to detect the visual angle which can be identified by the human eye, i.e. the visual resolution of the eye, not the accommodation of the crystalline lens. The emmetropic eye requires moderate accommodation to view an object clearly at some distance. The moderate accommodation refers to the variation range of the accommodation of the crystalline lens when the eye views the object at some distance. The lens has a moderate accommodation of 100 degrees when the distance varies from 1 meter to infinity. The accommodation of the crystalline lens decreases slowly when the distance between the eye and the object is more than 1 meter. The accommodation of the crystalline lens decreases by 80 degrees when the distance varies from 1 meter to 5 meters and decreases by 100 degrees in total when the distance varies from 1 meter to infinity, which reaches the limitation. This means that 5 meters are a critical value. The accommodation of the crystalline lens may not be influenced much by the distance beyond 5 meters. Thus, the distance between the subject and the eye chart is 5 meters during the visual acuity measurements.

The indicative line on the device points to a scale mark indicating zero on a dial and the eye chart is located at the focal point of the imaging lens group during the visual acuity measurements of the naked eye. The visual acuity value corresponding to the smallest optotype which can be identified by the subject viewing the eye chart is the visual acuity of the naked eye. Actually, the distance between the eye and the eye chart should be infinity during the visual acuity measurements, but according to the national standard, the distance is stipulated to be 5 meters, because infinity is impracticable and the diopter difference between 5 meters and infinity is 0.2D. The technical solution adopted by the present invention is that the eye chart is located at the focal point of the imaging lens group during the visual acuity measurements of the naked eye, which is equivalent to setting the distance between the eye and the eye chart to be infinity, thereby meeting the real requirement of the distance between the eye and the eye chart during the visual acuity measurements. Therefore, when the portable vision detecting device according to the present invention is used to detect the vision of the human eye, the result of the detection will be more precise than that obtained by the method of setting the distance between the human eye and the eye chart to be 5 meters.

2. It is unnecessary to wear corrected lens during the best corrected visual acuity (fully corrected) measurements, and the result thereof is equivalent to the corrected visual acuity result measured when the scale mark indicating zero of the hand-held vision detecting device is rotated to the indicative line and the corrected lens with right strength is worn. The unique design of the present invention is to simplify the procedure of the corrected visual acuity measurements.

According to the traditional method of the best corrected visual acuity measurements, the subject should wear the corrected lens to perform the visual acuity measurements on the basis of the visual acuity measurements of the naked eye. The visual acuity value corresponding to the line of the smallest E optotypes in the eye chart of which the opening orientations can be seen clearly is the best corrected visual acuity, and the degrees of the corrected lens worn by the subject at this moment are the degrees of the lens to be worn. When the best corrected visual acuity is measured with the portable vision detecting device according to the present invention, it is unnecessary to wear the corrected lens, and it is just required to locate the cornea of the naked eye at the focal point at one side of the imaging lens group, then rotate the adjusting knob until the opening orientations of the line of the smallest E optotypes in the eye chart can be seen clearly. At this moment, the visual acuity value marked at both sides of the line of the smallest E optotypes is the best corrected visual acuity value, and the diopter value on the dial pointed to by the indicative line on the outside of the detecting device is the corrected visual acuity value which should be worn by the subject. For example, if the diopter value pointed to by the indicative line is +3D indicating the hyperopia, the strength of the glasses which should be worn by the subject is hyperopic 300 degrees, and if the diopter value pointed to by the indicative line is -3D indicating the myopia, the strength of the glasses which should be worn by the subject is myopic 300 degrees. The "D" used herein represents the diopter, and 1 diopter equals 100 degrees; "+" indicates hyperopia, "-" indicates myopia. Therefore, with the hand-held vision detecting device according to the present invention, the visual acuity measurements and correction may be directly performed and the strength of the glasses which should be worn may be determined, without wearing glasses.

Now, it is required to prove that the corrected visual acuity measured by rotating the scale mark indicating zero of the hand-held vision detecting device to the indicative line when a suitable glasses is worn is equal to the best corrected visual acuity measured by the above mentioned method (the dial is rotated until the eye chart can be seen most clearly, and the visual acuity corresponding to the smallest E optotypes in the eye chart which can be seen clearly is the best corrected visual acuity; at this moment, the value corresponding to the dial is the strength of the glasses which should be worn).

At first, the diopter of both is proved to be equal (i.e., the divergence of the light incident on the human eye is equal). Because the optometry function of the device is designed according to this requirement, that is to say, the diopter on the dial of the device meets the requirement in design, it is unnecessary to prove it.

Assuming the width of a stroke in the eye chart of the hand-held vision detecting device to be $h_0$, the focal length of the imaging lens group of the hand-held vision detecting device to be $f_0$, the focal length of the human eye to be $f_e$, if the human eye is emmetropic, the visual angle $\alpha$ is:

$$\alpha = \frac{h_0}{f_0} = \frac{h}{f_e}$$

The width of the image formed from the stroke in the eye chart on the retina is:

$$h = \frac{f_e h_o}{f_0}$$

If the human eye is ametropic, assuming the corresponding optometry value is D, the eye axis length thereof $L$ is:

$$L = \frac{1}{D + \dfrac{1}{f_e}}$$

If the glasses are worn, the width of the image formed on the retina is:

$$h' = \frac{L h_0}{f_0} = \frac{h_0}{f_0 (D + \dfrac{1}{f_e})}$$

If the glasses are not worn and the hand-held vision detecting device is adjusted to the place where the scale mark is $D$, the displacement distance of the eye chart of the hand-held vision detecting device is:

$x = D f_0{}^2$

Regarding the imaging lens group of the hand-held vision detecting device and the human eye as a combination with a distance therebetween of $f_0$, the focal length of the combined imaging lenses is still $f_0$ based on calculation. According the formula of the combined imaging lenses,

$$\Delta = f_0 - f_0 - f_e = -f_e$$

$$x_F = \frac{-f_0^2}{\Delta} = \frac{f_0^2}{f_e}$$

$$x'_F = \frac{f_e^2}{\Delta} = -f_e$$

could be obtained.

In the above formulas, $\Delta$ is the distance between the inner focal points of the two lenses, and XF and XF' are the distances between the two focal points at both sides of the combined lenses and the two lenses, respectively.

The distance of the left principal plane of the combined imaging lenses relative to the imaging lens group of the hand-held imaging lens group is:

$$-f_0 + x_F + f_0 = x_F = \frac{f_0^2}{f_e}$$

The distance of the right principal plane of the combined imaging lenses relative to the surface of the human eye is:

$$f_e + x'_F - f_0 = -f_0$$

That is to say, the right principal plane of the combined imaging lenses is kept stationary at the lens of the hand-held vision detecting device.

The visual angle relative to the combined imaging lenses is:

$$\alpha' = \frac{h_0}{f_0 + x + \dfrac{f_0^2}{f_e}} = \frac{h''}{f_e + f_0}$$

The width of the image formed on the retina is:

$$h'' = \frac{h_0(f_e + f_0)}{f_0 + x + \dfrac{f_0^2}{f_e}} = \frac{h_0(f_e + f_0)}{f_0 + Df_0^2 + \dfrac{f_0^2}{f_e}}$$

The size ratio of the image viewed with wearing the glasses to that viewed without wearing the glasses is:

$$h' : h'' = \frac{h_0}{f_0(D + \dfrac{1}{f_e})} : \frac{h_0(f_e + f_0)}{f_0 + Df_0^2 + \dfrac{f_0^2}{f_e}} = \frac{1 + Df_0 + \dfrac{f_0}{f_e}}{(f_e + f_0)(D + \dfrac{1}{f_e})} = \frac{1 + Df_0 + \dfrac{f_0}{f_e}}{1 + Df_0 + \dfrac{f_0}{f_e} + Df_e}$$

When $f_0$ is 40 mm and $f_e$ is 17 mm, as for myopic 300 degrees, the above formula equals 1.02, that is to say, the size difference is 2%. As for myopic 1000 degrees, the above formula equals 1.06, that is to say, the size difference is 6%. Thus, these differences may be substantially ignored.

This proves that the size of the eye chart viewed with these two methods is substantially equal. Therefore, the corrected visual acuity measured by rotating the scale mark indicating zero of the hand-held vision detecting device to the indicative line when a suitable glasses is worn is equal to the best corrected visual acuity measured by the above mentioned method (the dial is rotated until the eye chart can be seen most clearly, and the visual acuity corresponding to the smallest E optotypes in the eye chart which can be seen clearly is the best corrected visual acuity; at this moment, the value corresponding to the dial is the strength of the glasses which should be worn).

A hand-held vision detecting device, comprises an eye chart and an imaging lens group consisting of at least one imaging lens, wherein the center of the eye chart is movably provided on the optical axis at one side of the imaging lens group and can move along the optical axis, and the cornea of the subject is located on the optical axis at the other side of the imaging lens group. The functions of the vision detection and correction can be realized by the present hand-held vision detecting device. The various functions of the vision detection and correction mentioned herein are depicted in an explanation hereinafter for the vision detection and operating steps.

Example 1

The example 1 is directed to the case in which the cornea of the subject is located at the focal point at the other side of the imaging lens group of the hand-held vision detecting device.

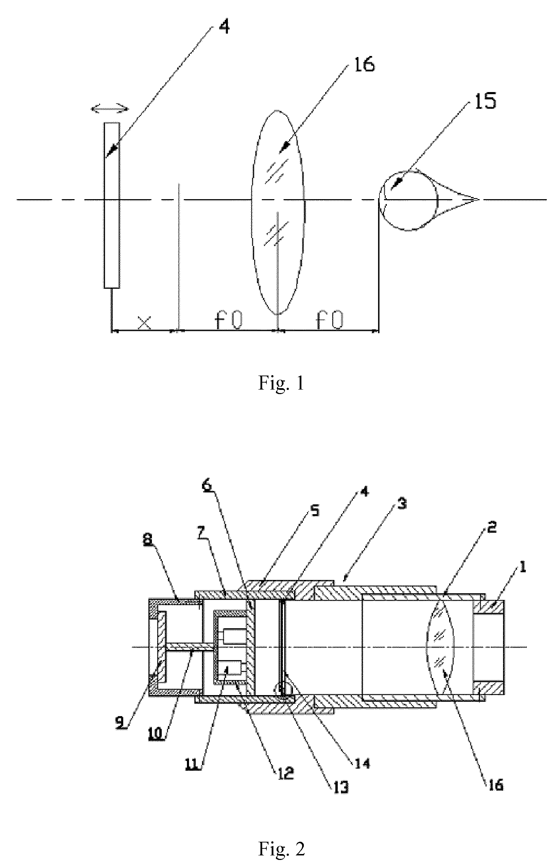

Referring to Fig. 1, Fig. 1 is the schematic structural view of the optical path of the present invention, which includes an imaging lens group 16 and an eye chart 4. The cornea of the subject is located at the focal point at one side of the imaging lens group 16. The center of the eye chart 4 is provided at the other side of the imaging lens group 16 and can move forwards and backwards along the optical path. The purpose of locating the cornea of the subject at the focal point at one side of the imaging lens group 16 is to achieve the linear scaling of the diopter. In the formula $D1 = x /(f_0 * f_0)$, $D1$ is the diopter value of the human eye, and $x$ is a distance from the eye chart 4 to the focal point $f_0$ of the lens group 16, wherein $x$ is positive if the eye chart is away from the imaging lens group 16 and $x$ is negative if the eye chart is close to the imaging lens group 16. Specifically, if the eye chart 4 moves to the focal point at the other side of the imaging lens group 16, $x$ is zero, and D1 is zero according to the formula $D1 = x /(f_0 * f_0)$, that is to say, the diopter value is zero. When the optotypes in the eye chart 4 is viewed in the case that the diopter value is zero, the visual acuity value marked at both sides of the smallest optotypes of which the opening orientations can be identified is the visual acuity value of the naked eye. At this moment, the cornea 15 of the human eye and the center of the eye chart 4 are located at the focal points at both sides of the imaging lens group 16, respectively.

The eye chart 4 continues to move forwards and backwards, until the subject can identify the opening orientations of the smallest optotypes in the eye chart 4, and thus the visual acuity value marked at both sides thereof is the corrected visual acuity value of the subject.

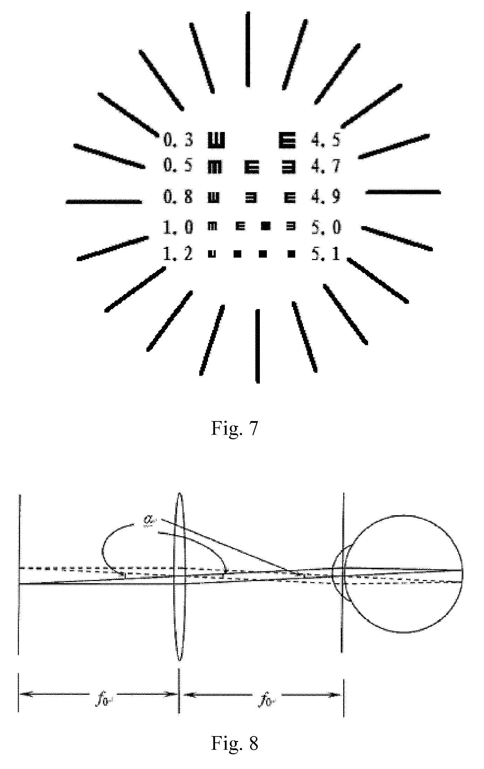

Referring to Fig. 8, it illustrates that an opening angle subtended by the width of the stroke at the center of a lens is equal to the visual angle of the human eye.

The eye chart 4 may be a common eye chart or a non-common eye chart. Specifically, the common eye chart is at least a tumbling E chart, a tumbling C chart or a tumbling letter chart, and of course, may be other types of the common eye charts. The width of the stroke of the optotypes in the eye chart used in the present invention is equal to the product of the focal length $f_0$ of the imaging lens group 16 and the visual angle $\alpha$. The present invention only describes the tumbling E chart as an embodiment, however, the usage of other types of eye charts other than the tumbling E chart in the present device is still within the scope of protection of the present invention.

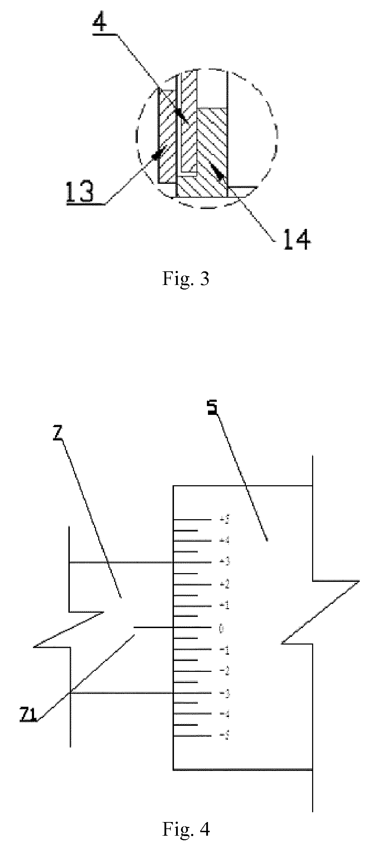

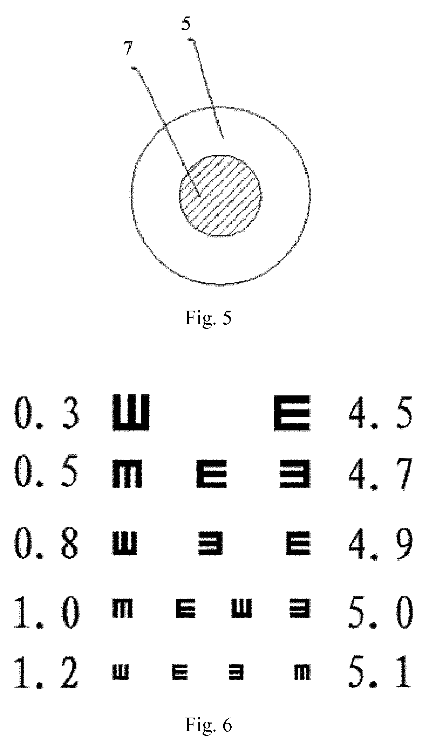

Referring to Fig. 2, Fig. 4 and Fig. 5, the hand-held vision detecting device further comprises a sleeve 7 provided in the middle of one end thereof and an adjusting knob 5 in which the sleeve 7 is sheathed as shown in Fig. 2. Referring to Fig. 4 and Fig. 5, an indicative line 71 is marked on the outer circumference of the sleeve 7; only the scale mark indicating zero may be marked on the outer circumference of the adjusting knob 5, or the scale marks indicating other diopter values including zero may also be uniformly marked on the outer circumference of the adjusting knob 5. When the indicative line 71 points to the scale mark indicating zero, the eye chart 4 and the cornea 15 of the human eye are just located at the focal points at both sides of the imaging lens group 16, respectively. If only the scale mark indicating zero is marked on the outer circumference of the adjusting knob 5, only the visual acuity of the naked eye and corrected visual acuity can be measured, but the diopter value corresponding to the corrected visual acuity cannot be measured, that is to say, the strength of the glasses cannot be known. If the scale marks indicating other linear diopter values including zero are marked on the outer circumference of the adjusting knob 5, the scale marks indicating other diopter values pointed to by the indicative line 7 are the strengths of the glasses which should be worn. "+" indicates hyperopia, and "-" indicates myopia, among these scale values. For example, during the rotation of the adjusting knob 5 when measuring the corrected visual acuity, if the indicative line 71 points to "+3", it is indicated that the diopter of the eye of the subject is hyperopic 300 degrees, and if the indicative line 71 points to "-3", it is indicated that the diopter of the eye of the subject is myopic 300 degrees. If the indicative line 71 points to a short scale mark between the scale marks with numbers, such as the short scale mark between the scale marks indicating "+1" and "+2", hyperopic 150 degrees is indicated. It should be noted that the range of the diopter indicated by the scale marks as shown in Fig. 2 is merely illustrative and not represents that the measuring range of the diopter of the present device is -500 degrees to +500 degrees.

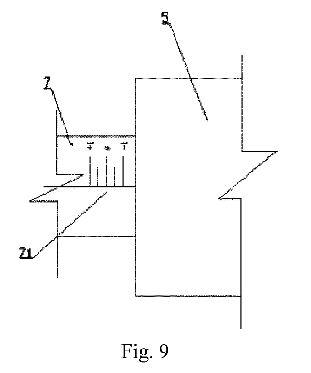

Furthermore, referring to Fig. 9, the scale mark indicating zero and the scale marks indicating other diopter values including zero may also be provided on the outer circumference of the sleeve 7 along the axial direction of the sleeve 7. At this moment, the scale mark on the sleeve 7 aligned with the end face of the outer circumference of the adjusting knob 5 is the diopter value, during the rotation of the adjusting knob 5. Similarly, the visual acuity of the naked eye can be measured when the adjusting knob is rotated to the scale mark indicating zero, and if the scale marks indicating other diopter values other than zero are marked on the sleeve 7, the corrected diopter value can be measured when the end face of the outer circumference of the adjusting knob 5 is adjusted to be aligned with the scale marks indicating corresponding diopter values.

Referring to Fig. 2 and Fig. 3, as mentioned above, the eye chart 4 can move along the optical path at one side of the imaging lens group 16. Specifically, in the present embodiment, the eye chart 4 is provided between an eye chart cover 13 and an eye chart fixing base 14. The eye chart fixing base 14 is fixedly provided on the interior side wall of the sleeve. The eye chart cover 13 covers the outer edge of the eye chart 4 and is detachably connected to the eye chart fixing base 14. The purpose of such configuration is to facilitate the replacement of the eye chart 4. The through hole through which the optical path can pass is provided in the middle of the eye chart cover 13 and the eye chart fixing base 14. Because the eye chart fixing base 14 is fixedly provided on the interior side wall of the sleeve 7, the sleeve 7 is driven to move forwards and backwards by the adjusting knob 5 during the rotation of the adjusting knob 5, and in turn the eye chart 4 is driven to move forwards and backwards.

The present vision detecting device further comprises a handle 3 provided in the middle thereof, and the shape of the handle 3 is a columnar structure with two substantially elliptical end faces and a through hole along the axis of the handle 3. The purpose of configurating the handle 3 to be elliptical is to facilitate gripping the present device by the subject, so as to meet ergonomic requirements. Of course, the handle may have other shapes other than the elliptical shape as long as the device is easily to be gripped. The adjusting knob 5 is detachably connected to one end of the handle 3. Specifically, in the present embodiment, a step at one end of the handle 3 is sheathed in the adjusting knob 5 and is connected thereto via a pin (not shown). The imaging lens group 16 is fixedly provided inside of an imaging lens group fixing base 2. The imaging lens group fixing base 2 together with the imaging lens group 16 is detachably connected to the other end of the handle 3. Specifically, in the present embodiment, the imaging lens group fixing base 2 is connected to the other end of the handle 3 by means of threads. Of course, other detachable connection means may also be deployed, such as rotatable gear connection and slot connection, which all falls within the scope of protection of the present invention. Because the imaging lens group fixing base 2 together with the imaging lens group 16 is connected to the other end of the handle 3 and cannot move forwards and backwards, this means that the imaging lens group 16 as shown in Fig. 1 is fixed stationarily in one place in the optical path. However, the eye chart 4 may be driven to move forwards and backwards in the optical path at one side of the imaging lens group 16 by rotating the adjusting knob 5. The design concept of such configuration is similar to the principle of optical path as shown in Fig. 1.

Referring to Fig. 2, the present vision detecting device further comprises a member providing an illuminating light source for the eye chart 4, and the illuminating light therefrom meets the luminance requirements stipulated in the standard GB11533-2011. Specifically, the member comprises an illuminating light source 6 and a battery support 12 which are fixedly provided inside of the fixing base 2 and behind the eye chart cover 13. Some batteries 11 are provided in the battery support 12. The batteries should be removed from the battery support 12 when unnecessary. The battery support 12 is connected to a button panel 10 which is connected to a button 9. The button panel 10 and the button 9 are both provided inside of a battery fixing base 8. The battery fixing base 8 is detachably connected to the sleeve 7. Specifically, in the present embodiment, the battery fixing base 8 is connected to the sleeve 7 by means of threads. The button 9 is equivalent to the switch of the present vision detecting device. The illuminating light source will be turned on by pressing the button 9 to provide the illuminating light for the eye chart 4 when the visual acuity is required to be measured, and the illuminating light source will be turned off by pressing the button 9 again when unnecessary.

Furthermore, the present vision detecting device may not comprise the member providing the illuminating light source for the eye chart 4, and at this moment, the vision detection and correction can be performed with natural light.

Referring to Fig. 2, an eye protecting ring 1 is further provided on the outer end of the imaging lens group fixing base 2. The distance between the eye protecting ring 1 and the imaging lens group fixing base 2 is constant, and the eye protecting ring 1 is used to improve the comfort of the user during the visual acuity measurements.

Moreover, it should be noted that the imaging lens group 16 of the present embodiment may be consist of one lens or a plurality of lenses. The imaging lens group 16 may be a single lens, a doublet lens or a lens group.

Example 2

The cornea of the eye is not required to be located at the focal point at one side of the imaging lens group, which is different from Example 1. In this situation, the diopter scale marked on the adjusting knob 5 is not linear, but all the functions of the hand-held vision detecting device of Example 1 can be still realized.

The invention further discloses the vision detecting function and the specific operating method which can be realized by the hand-held vision detecting device.

Please refer to Fig. 4 and Fig. 6 for the convenience of understanding, and it should be noted that the tumbling E chart as shown in Fig. 6 conforms to the international standard. The eye chart may be a tumbling C chart or other eye charts. The visual acuity value and the corresponding optotypes in the eye chart may be replaced or regulated under the international standard.

1. Visual acuity measurements of the naked eye

Referring to Fig. 4 and Fig. 6, the visual acuity value marked at both sides of the line of the smallest E optotypes in the tumbling E chart as shown in Fig. 6 of which the opening orientations can be identified is the visual acuity value of the naked eye when the scale mark indicating zero on the adjusting knob 5 is aligned with the indicative line 71.

2. Corrected visual acuity measurements

When the subject watches the tumbling E chart as shown in Fig. 6, the adjusting knob 5 is rotated until the smallest E optotypes can be identified, and the corresponding visual acuity value at both sides thereof is the best corrected visual acuity value. At this moment, the corresponding value in the diopter scale which the indicative line 71 is aligned with is the strength of the glasses which should be worn. For example, if the indicative line 7 is aligned with "-2", it means myopic 200 degree. Of course, only the best corrected visual acuity value can be measured if the diopter scale is not marked on the adjusting knob 5.

3. Measurements for detecting the strength of the glasses which should be worn when the corrected visual acuity is 1.0

The sleeve 7 is rotated until the device is extended to the maximum length, and then the adjusting knob 5 is rotated until the opening orientations of the E optotypes in the eye chart corresponding to the visual acuity value of 1.0 can be identified and the opening orientations of the E optotypes corresponding to the visual acuity value of 1.2 cannot be identified when the subject watches the tumbling E chart as shown in Fig. 6. At this moment, the scale value corresponding to the indicative line 71 as shown in Fig. 4 is the strength of the glasses which should be worn when the corrected visual acuity value is 1.0.

4. Poor vision and amblyopia vision detection

The patient who cannot identify the opening orientations of the E optotypes in the tumbling E chart as shown in Fig. 6 corresponding to the visual acuity value of 0.3 no matter how the adjusting knob 5 is rotated is a patient with poor vision.
The patient who cannot identify the opening orientations of the E optotypes in the tumbling E chart as shown in Fig. 6 corresponding to the visual acuity value of 1.0 (but can identify the opening orientations of the E optotypes corresponding to the visual acuity value of 0.8 at most) no matter how the adjusting knob 5 is rotated is a patient with amblyopia vision.
In the various functional operation realized by the above vision detecting device, the term "clearly" means that not only the opening orientations of the E optotypes can be seen clearly, but also the strokes of the E optotypes can be seen clearly. The term "identify" means that the whole E optotypes may not be seen clearly, but the opening orientations of the E optotypes can be determined. Moreover, the width of the strokes of the E optotypes in the above eye chart is equal to the product of the focal length $f_0$ of the imaging lens group and the visual angle $\alpha$. Besides, the width of the strokes of the optotypes in the eye chart used in the present device is equal to the product of the focal length $f_0$ of the imaging lens group and the visual angle $\alpha$.
Referring to Fig. 7, the eye chart 4 may also have a pattern including a peripheral astigmatic chart and an inner eye chart. At this moment, not only the function of eye chart, but also the functions of astigmatism detection and eye adjustment training can be realized in the eye chart 4 with the present device.
The above are the preferred embodiments of the present invention. It should be noted that for those skilled in the art, a number of improvements and changes can be made without departing from the spirit of the present invention. Those improvements and changes can also be considered falling within the scope of protection of the present invention.

**Claims**

1. A hand-held vision detecting device, comprising an eye chart and an imaging lens group consisting of at least one imaging lens, wherein the cornea of a subject is located on the optical axis at one side of the imaging lens group, and the center of the eye chart is movably provided on the optical axis at the other side of the imaging lens group and can move forwards and backwards along the optical axis.

2. The hand-held vision detecting device according to claim 1, wherein the cornea of the subject is located at the focal point at one side of the imaging lens group.

3.  The hand-held vision detecting device according to claim 2, wherein the center of the eye chart is movably provided at the focal point at the other side of the imaging lens group.

4.  The hand-held vision detecting device according to claim 2 or 3, further comprising a sleeve provided in the middle of one end thereof and an adjusting knob in which the sleeve is sheathed, wherein an indicative line is marked on the outer circumference of the sleeve; the eye chart is provided inside of the sleeve and can move away from or close to the imaging lens group along with the sleeve during the rotation of the adjusting knob; a scale mark indicating a diopter value is zero is marked on the outer circumference of the adjusting knob, or the scale mark indicating the diopter value is zero is marked on the outer circumference of the sleeve along its axis; the eye chart is just located at the focal point at the other side of the imaging lens group when the diopter value is zero.

5.  The hand-held vision detecting device according to claim 4, wherein scale marks indicating other diopter values D1 are marked on the outer circumference of the adjusting knob or the outer circumference of the sleeve along the axis of the sleeve, and D1 meets the formula: $D1 = x / (f_0 * f_0)$,
    wherein x is a distance from the eye chart to the focal point at the other side of the imaging lens group, and x is positive if the eye chart is away from the imaging lens group, and is negative if the eye chart is close to the imaging lens group.

6.  The hand-held vision detecting device according to claim 4 or 5, further comprising an eye chart fixing base and a eye chart cover, wherein the eye chart fixing base is fixedly provided on the interior side wall of the sleeve; the eye chart is provided between the eye chart fixing base and the eye chart cover; a through hole through which illuminating light or natural light can pass is provided in the middle of the eye chart fixing base and the eye chart cover.

7.  The hand-held vision detecting device according to any one of claims 4-6, further comprising an illuminating light source and a battery support which are fixedly provided in sequence inside of the sleeve and behind the eye chart cover, wherein a button panel component and a button are provided in sequence behind the battery support; the button panel component and the button are provided inside of a battery fixing base; the battery fixing base is detachably connected to the sleeve.

8.  The hand-held vision detecting device according to any one of claims 4-7, further comprising a handle provided in the middle thereof, wherein the handle is hollow, and two ends of the handle are detachably connected to the adjusting knob and an imaging lens group fixing base, respectively; the imaging lens group is fixed inside of the imaging lens group fixing base.

9.  The hand-held vision detecting device according to claim 8, wherein an eye protecting ring is connected to an outer end of the imaging lens group fixing base.

10. The hand-held vision detecting device according to any one of claims 1-9, wherein the imaging lens group is a single lens, a doublet lens or a lens group.

11. The hand-held vision detecting device according to any one of claims 1-9, wherein the eye chart is at least a common eye chart.

12. The hand-held vision detecting device according to claim 11, wherein the common eye chart is at least a tumbling E chart, a tumbling C chart or a tumbling letter chart.

13. The hand-held vision detecting device according to any one of claims 1-12, wherein the eye chart is further provided with an astigmatism chart.

14. A vision detecting method for detecting the visual acuity of naked eye with a hand-held vision detecting device, comprising:

    aligning the location of a scale mark indicating zero on an adjusting knob with an indicative line;
    keeping the location of the scale mark indicating zero stationary, wherein the visual acuity value marked at both sides of the smallest optotypes which can be identified by a subject and the width of strokes of which is equal to the product of the focal length $f_0$ of an imaging lens group and a visual angle $\alpha$ is the visual acuity of the naked eye.

15. A vision detecting method for detecting corrected visual acuity with a hand-held vision detecting device, comprising:

rotating a adjusting knob until an eye chart being watching is clearest, wherein the visual acuity value marked at both sides of the smallest optotypes which can be identified and the width of strokes of which is equal to the product of the focal length $f_0$ of an imaging lens group and a visual angle $\alpha$ is the best corrected visual acuity; determining a diopter scale mark corresponding to an indicative line, wherein a diopter value corresponding to the scale mark is the strength of the glasses which should be worn by the subject.

16. A vision detecting method for detecting poor vision with a hand-held vision detecting device, comprising:

a subject is judged as a patient with poor vision if optotypes in an eye chart which are corresponding to the visual acuity value of 0.3 and the width of strokes of which is equal to the product of the focal length $f_0$ of an imaging lens group and a visual angle $\alpha$ cannot be identified by the subject no matter how an adjusting knob is rotated clockwise or anticlockwise.

17. A vision detecting method for detecting amblyopia vision with a hand-held vision detecting device, comprising:

a subject is judged as a patient with amblyopia vision if optotypes in an eye chart which are corresponding to the visual acuity value of 1.0 and the width of strokes of which is equal to the product of the focal length $f_0$ of an imaging lens group and a visual angle $\alpha$ cannot be identified by the subject no matter how an adjusting knob is rotated clockwise or anticlockwise by the subject.

Fig. 1

Fig. 2

Fig. 3

Fig. 4

Fig. 5

| | | | |
|---|---|---|---|
| 0. 3 | Ш | E | 4. 5 |
| 0. 5 | m | E ∃ | 4. 7 |
| 0. 8 | Ш | ∃ E | 4. 9 |
| 1. 0 | m E Ш ∃ | | 5. 0 |
| 1. 2 | Ш E ∃ m | | 5. 1 |

Fig. 6

Fig. 7

Fig. 8

Fig. 9

# INTERNATIONAL SEARCH REPORT

| International application No. |
|---|
| **PCT/CN2014/086791** |

**A.  CLASSIFICATION OF SUBJECT MATTER**

A61B 3/036 (2006.01) i

According to International Patent Classification (IPC) or to both national classification and IPC

**B.  FIELDS SEARCHED**

Minimum documentation searched (classification system followed by classification symbols)

IPC: A61B 3/-

Documentation searched other than minimum documentation to the extent that such documents are included in the fields searched

Electronic data base consulted during the international search (name of data base and, where practicable, search terms used)

CNPAT, CNKI, WPI, EPODOC: SHENZHEN CERTAINN TECHNOLOGY CO., LTD.; SHENZHEN MOPTIM IMAGING TECHNIQUE CO., LTD.; WANG, Hui; ZHEN, Yi; GUO, Shuguang; HE, Weihong; LUAN, Dun; LI, Peng; eye chart, optometry, eyesight, turntable, lens, eye, knob, adjust+

**C.  DOCUMENTS CONSIDERED TO BE RELEVANT**

| Category* | Citation of document, with indication, where appropriate, of the relevant passages | Relevant to claim No. |
|---|---|---|
| PX | CN 103654708 A (SHENZHEN MOPTIM IMAGING TECHNIQUE CO., LTD.), 26 March 2014 (26.03.2014), claims 1-13 | 1-13 |
| PX | CN 203591249 U (SHENZHEN MOPTIM IMAGING TECHNIQUE CO., LTD.), 14 May 2014 (14.05.2014), claims 1-13 | 1-13 |
| X | CN 103300813 A (SHENZHEN MOPTIM IMAGING TECHNIQUE CO., LTD. et al.), 18 September 2013 (18.09.2013), description, paragraphs [0077]-[0081] and [0142], claim 1, and figures 1-2 | 1-13 |
| A | CN 1843287 A (ZHANG, Huanxu et al.), 11 October 2006 (11.10.2006), the whole document | 1-13 |
| A | CN 1964687 A (MATSUSHITA ELECTRIC INDUSTRIAL CO., LTD. et al.), 16 May 2007 (16.05.2007), the whole document | 1-13 |
| A | US 4148565 A (GUNST, O.), 10 April 1979 (10.04.1979), the whole document | 1-13 |

☐ Further documents are listed in the continuation of Box C.     ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 02 December 2014 (02.12.2014) | **23 December 2014 (23.12.2014)** |

| Name and mailing address of the ISA/CN:<br>State Intellectual Property Office of the P. R. China<br>No. 6, Xitucheng Road, Jimenqiao<br>Haidian District, Beijing 100088, China<br>Facsimile No.: (86-10) 62019451 | Authorized officer<br><br>**LI, Yinqin**<br><br>Telephone No.: (86-10) **61648460** |

Form PCT/ISA/210 (second sheet) (July 2009)

## INTERNATIONAL SEARCH REPORT

| International application No. |
| --- |
| **PCT/CN2014/086791** |

**Box No. II    Observations where certain claims were found unsearchable (Continuation of item 2 of first sheet)**

This international search report has not been established in respect of certain claims under Article 17(2)(a) for the following reasons:

1. ☒   Claims Nos.: 14-17
      because they relate to subject matter not required to be searched by this Authority, namely:
    [1] Claims 14-17 relate to a method of testing eyesight, which belongs to a diagnostic method implemented on a living human or animal body, and thus do not meet the requirements as defined in PCT Rule 39.1.

2. ☐   Claims Nos.:
      because they relate to parts of the international application that do not comply with the prescribed requirements to such an
      extent that no meaningful international search can be carried out, specifically:

3. ☐   Claims Nos.:
      because they are dependent claims and are not drafted in accordance with the second and third sentences of Rule 6.4(a).

Form PCT/ISA/210 (continuation of first sheet (2)) (July 2009)

## INTERNATIONAL SEARCH REPORT
### Information on patent family members

| International application No. |
|---|
| **PCT/CN2014/086791** |

| Patent Documents referred in the Report | Publication Date | Patent Family | Publication Date |
|---|---|---|---|
| CN 103654708 A | 26 March 2014 | None | |
| CN 203591249 U | 14 May 2014 | None | |
| CN 103300813 A | 18 September 2013 | None | |
| CN 1843287 A | 11 October 2006 | CN 100427024 C | 22 October 2008 |
| CN 1964687 A | 16 May 2007 | WO 2006006563 A1 | 19 January 2006 |
| | | US 2009168016 A1 | 02 July 2009 |
| | | JPWO 2006006563 A1 | 24 April 2008 |
| US 4148565 A | 10 April 1979 | None | |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 99243321 A
- WO 201020230301 A
- GB 115332011 A